# EUROPEAN PATENT SPECIFICATION

(11) **EP 0 508 324 B1**
(45) Date of publication and mention of the grant of the patent: **17.03.1999**
(21) Application number: 92105805.3
(22) Date of filing: 03.04.1992
(51) Int. Cl.: A61K 7/48, A61K 7/06, A61K 7/04, A61K 31/19

(54) **Use of cosmetic compositions comprising 2-hydrocarboxylic acids and related compounds for alleviating signs of nail changes**
Verwendung von 2-Hydroxycarbonsäure oder deren Derivaten enthaltende Zusammensetzung zur Linderung von Wechseln Nagelveränderungen
Utilisation des compositions cosmétiques contenant des acides 2-hydroxycarboxyliques et dérivées pour atténuer les signes d'altération des ongles

(30) Priority: 10.04.1991 US 683437
(43) Date of publication of application: 14.10.1992
(73) Proprietor: Yu, Ruey J., Dr., Ambler Pennsylvania 19002 (US); Van Scott, Eugene J., Dr., Abington Pennsylvania 19001 (US)
(72) Inventor: Yu, Ruey J., Dr., Ambler Pennsylvania 19002 (US); Van Scott, Eugene J., Dr., Abington Pennsylvania 19001 (US)
(74) Representative: Olgemöller, Luitgard, Dr.

(56) References cited:
- EP-A- 232 982
- EP-A- 273 202
- EP-A- 403 304
- EP-A- 413 528

## Description

### FIELD OF THE INVENTION

This application relates to topical compositions containing a 2-hydroxycarboxylic acid or a related compound for use in alleviating or improving the dermatological signs of nail associated with intrinsic aging, as well as changes or damage caused by extrinsic factors such as sunlight, radiations, air pollution, wind, cold, heat, dampness, chemicals, smoke, and cigarette smoking.

### BRIEF DESCRIPTION OF THE PRIOR ART

In our U.S.Patent No. 3,879,537 entitled "Treatment of Ichthyosiform Dermatoses" we described and claimed the use of topical compositions containing an alpha hydroxyacid to alleviate the symptoms of ichthyosis. In our U.S.Patent No. 3,920,835 entitled "Treatment of Disturbed Keratinization" we described and claimed the use of topical compositions containing an alpha hydroxyacid to alleviate the symptoms of acne. In our U.S.Patent No. 3,984,566 entitled "Method of Alleviating the Symptoms of Dandruff" we described and claimed the use of topical compositions containing an alpha hydroxyacid to improve the symptoms of dandruff.

In our U.S.Patent No. 4,105,783 entitled "Therapeutic Treatment of Dry Skin"; U.S.Patent No. 4,197,316 entitled "Treatment of Dry Skin"; and U.S.Patent No. 4,380,549 entitled "Topical Treatment of Dry Skin"; we described and claimed the use of topical compositions containing an alpha hydroxyacid to alleviate or improve the symptoms of dry skin. In our U.S.Patent No. 4,234,599 entitled "Treatment of Skin Keratoses with Alpha Hydroxyacids and Related Compounds", we described and claimed the use of topical compositions containing an alpha hydroxyacid or the related compound to alleviate the symptoms of actinic or nonactinic skin keratoses. In our U.S.Patent No. 4,363,815 entitled "Alpha Hydroxyacids, Alpha Ketoacids and Their Use in Treating Skin Conditions", we described and claimed the use of topical compositions containing certain alpha hydroxyacids or the related compounds to improve skin conditions characterized by inflammation or disturbed keratinization.

In a report entitled "Topical Tretinoin for Photoaged Skin" by Albert M. Kligman, Gary L. Grove, Ryoji Hirose and James J. Leyden published in J. American Academy of Dermatology Vol.15, pages 836-859, 886-887, 1986, daily topical application of 0.05% tretinoin (also known as all-trans retinoic acid) in a cream has been found to improve photodamaged skin. In another report entitled "Topical Tretinoin Improves Photoaged Skin: A Double-blind Vehicle-controlled Study" by Jonathan S. Weiss, Charles N. Ellis, John T. Headington, Theresa Tincoff, Ted A. Hamilton and John J. Voorhees published in J American Medical Association Vol. 259 pages 527-532, 1988, daily topical application of 0.1% tretinoin as compared to vehicle alone application for 16 weeks has been shown to improve photoaged skin. One side-effect has been a dermatitis encountered by 92% of the patients participating in this study. The dermatitis was characterized by a patchy erythema, localized swelling, dry skin, and mild scaling. Patients complained about burning, tingling, or pruritus. In yet another report entitled "Topical Tretinoin in the Treatment of Aging Skin" by Jonathan S. Weiss, Charles N. Ellis, John T. Headington and John J. Voorhees published in J. American Academy of Dermatology Vol.19, pages 169-175, 1988, topical application of 0.1% tretinoin cream for 8 to 12 months has been found to improve clinical signs of aging skin. The side effects have been burning sensation in the eyes and mild skin irritations.

US Parent application Serial No. 07/469,738 described in addition to the main subject certain compositions containing hydroxycarboxylic acids and the related ketocarboxylic acids for topical treatment of wrinkles and skin changes associated with aging. The related US application of Serial No. 07/393,749 (the priority of which is claimed in EP-A-0 413 528) described in addition to the main subject a topical treatment to alleviate or remedy warts, nail infections, age spots, wrinkles and aging related skin changes with a composition containing certain alpha hydroxyacids or the related compounds.

A fungal infection, however, cannot be regarded as being "intrinsic or extrinsic aging".

EP-A-273202 is directed to additives enhancing topical actions of therapeutic agents. The compositions are cosmetic or therapeutic dermatological agents, the aim of the use is to improve the skin. Likely, the therapeutic potentials of alpha hydroxy acids in this regard are also described in the Canadian Journal of Dermatology, volume 1 (5), pp. 108 to 112.

EP-A-403304 discloses a shampoo composition, containing charged surfactant, which also contains 2-hydroxy alkanoic acid and a buffering agent which enhances uptake of the 2-hydroxy alkanoic acid. Use of this composition gives the benefit of increasing elasticity of the hair.

### SUMMARY OF THE INVENTION

Accordingly, it is an object of this invention to provide methods and compositions which can alleviate signs of nail changes associated with intrinsic and/or extrinsic aging.

We have now discovered that 2-hydroxycarboxylic acids and related compounds have unusual qualities which have not been disclosed in the prior art. Topical applications of compositions containing a 2-hydroxycarboxylic acid or a related compound have been found to improve cosmetic as well as clinical signs of changes in nails associated with intrinsic aging, or the damages caused by extrinsic factors such as sunlight, radiations, air pollution, wind, cold, dampness, heat, chemicals, smoke, and cigarette smoking. The signs of nail changes associated with intrinsic aging and the damages caused by extrinsic factors include thinning, fragility, splitting, lack of luster, uneven surface, and loss of flexibility and elasticity. 2-Hydroxycarboxylic acids and their related compounds which are useful for topical treatment of nail changes associated with intrinsic and/or extrinsic aging include, *inter alia*, 2-hydroxyethanoic acid, 2-hydroxypropanoic acid, 2-methyl 2-hydroxypropanoic acid, 2-phenyl 2-hydroxyehtanoic acid, 2-phenyl 2-methyl 2-hydroxyethanoic acid, 2-phenyl 3-hydroxypropanoic acid, 2,2-diphenyl 2-hydroxyethanoic acid, 2-hydroxybutane-1,4-dioic acid, 2,3-dihydroxybutane-1,4-dioic acid, 2-carboxy 2-hydroxypentane-1,5-dioic acid, 2-ketopropanoic acid, methyl 2-ketopropanoate, ethyl 2-ketopropanoate, and gluconolactone.

Additional objects and advantages of the invention will be set forth in part in the description that follows, and in part will be obvious from the description, or may be learned by practice of the invention. The objects and the advantages of this invention may be realized and obtained by means of the compositions and methods particularly pointed out in the appended claims.

### DETAILED DESCRIPTION OF THE PREFERRED EMBODIMENTS

Cutaneous aging is associated with intrinsic factors with or without the additional factors of extrinsic origin. The intrinsic aging is due to internal physiologic functions and is an inherent aging process of living beings, which has not been reversible nor preventable. Extrinsic aging, on the other hand, is due to external factors such as sunlight, radiations, air pollution, wind, cold, dampness, heat, chemicals, smoke, and cigarette smoking. A modification, improvement or alleviation of the signs associated with the intrinsic or extrinsic aging of nails is also now possible in accordance with this invention. Moreover, in some cases, it may be possible to eradicate such signs of intrinsic and extrinsic aging.

The signs of nail changes associated with intrinsic aging and the damages caused by extrinsic factors include thinning of nail plate; lack of luster, and uneven surface of nails; fragility and splitting of nails; and reduction of flexibility, resiliency, and elasticity of nails.

We have now discovered that use of cosmetic compositions containing 2-hydroxycarboxylic acid or related compounds are effective in alleviating signs of nail changes associated with intrinsic and/or extrinsic aging.

For convenience, the 2-hydroxycarboxylic acids and related compounds which may be used in accordance with this invention may be classified into three groups, namely (1) 2-hydroxycarboxylic acids, (2) 2-ketocarboxylic acids and esters thereof, and (3) other related compounds. The related compounds may include hydroxycarboxylic acids with the hydroxyl group at any position other than position 2, for example position 3, position 4 or position 5, as well as cyclic hydroxycarboxylic acids (e.g., ascorbic acid and quinic acid), and also may include ketocarboxylic acids and esters thereof. Preferred related compounds include 3-hydroxycarboxylic acids, and 2-ketocarboxylic acids and esters thereof.

### Group 1

The first group comprises organic carboxylic acids in which one hydroxy group is attached to the 2 position carbon atom of the acid. The generic structure of such 2-hydroxycarboxylic acids may be represented as follows:
(Rₐ) (R_{b}) C (OH) COOH
Where Rₐ and R_{b} may be the same or different and are independently selected from H, F, Cl, Br, alkyl, aralkyl or aryl group of saturated or unsaturated, isomeric or non-isomeric, straight or branched chain or cyclic form, having 1 to 29 carbon atoms, and in addition Rₐ and R_{b} may carry OH, CHO, COOH and alkoxy group having 1 to 9 carbon atoms. 2-Hydroxycarboxylic acids may be present as a free acid or lactone form, or in a salt form with an organic base or an inorganic alkali. 2-Hydroxycarboxylic acids may exist as stereoisomers as D, L, and DL forms when Rₐ and R_{b} are not identical.

Typical alkyl, aralkyl and aryl groups for Rₐ and R_{b} include methyl, ethyl, propyl, isopropyl, butyl, pentyl, octyl, decyl, dodecyl, hexadecyl, benzyl, and phenyl, etc. 2-Hydroxycarboxylic acids of the first group may be further divided into subgroups comprising (1) alkyl hydroxycarboxylic acids, (2) aralkyl and aryl hydroxycarboxylic acids, (3) polyhydroxy-carboxylic acids, and (4) hydroxy-polycarboxylic acids. The following are representative 2-hydroxycarboxylic acids in each subgroup.

### (1) Alkyl Hydroxycarboxylic Acids

1. 2-Hydroxyethanoic acid (Glycolic acid, hydroxyacetic acid)
   (H) (H) C (OH) COOH
2. 2-Hydroxypropanoic acid (Lactic acid)
   (CH₃) (H) C (OH) COOH
3. 2-Methyl 2-hydroxypropanoic acid (Methyllactic acid)
   (CH₃) (CH₃) C (OH) COOH
4. 2-Hydroxybutanoic acid
   (C₂H₅) (H) C (OH) COOH
5. 2-Hydroxypentanoic acid
   (C₃H₇) (H) C (OH) COOH
6. 2-Hydroxyhexanoic acid
   (C₄H₉) (H) C (OH) COOH
7. 2-Hydroxyheptanoic acid
   (C₅H₁₁) (H) C (OH) COOH
8. 2-Hydroxyoctanoic acid
   (C₆H₁₃) (H) C (OH) COOH
9. 2-Hydroxynonanoic acid
   (C₇H₁₅) (H) C (OH) COOH
10. 2-Hydroxydecanoic acid
   (C₈H₁₇) (H) C (OH) COOH
11. 2-Hydroxyundecanoic acid
   (C₉H₁₉) (H) C (OH) COOH
12. 2-Hydroxydodecanoic acid (Alpha hydroxylauric acid)
   (C₁₀H₂₁) (H) C (OH) COOH
13. 2-Hydroxytetradecanoic acid (Alpha hydroxymyristic acid)
   (C₁₂H₂₅) (H) C (OH) COOH
14. 2-Hydroxyhexadecanoic acid (Alpha hydroxypalmitic acid)
   (C₁₄H₂₉) (H) C (OH) COOH
15. 2-Hydroxyoctadecanoic acid (Alpha hydroxystearic acid)
   (C₁₆H₃₃) (H) C (OH) COOH
16. 2-Hydroxyeicosanoic acid (Alpha hydroxyarachidonic acid)
   (C₁₈H₃₇) (H) C (OH) COOH
17. 2-Hydroxytetraeicosanoic acid (Cerebronic acid)
   (C₂₂H₄₅) (H) C (OH) COOH
18. 2-Hydroxytetraeicosenoic acid (Alpha hydroxynervonic acid)
   (C₂₂H₄₃) (H) C (OH) COOH

### (2) Aralkyl And Aryl 2-Hydroxycarboxylic Acids

1. 2-Phenyl 2-hydroxyethanoic acid (Mandelic acid)
   (C₆H₅) (H) C (OH) COOH
2. 2,2-Diphenyl 2-hydroxyethanoic acid (Benzilic acid)
   (C₆H₅) (C₆H₅) C (OH) COOH
3. 3-Phenyl 2-hydroxypropanoic acid (Phenyllactic acid)
   (C₆H₅CH₂) (H) C (OH) COOH
4. 2-Phenyl 2-methyl 2-hydroxyethanoic acid (Atrolactic acid)
   (C₆H₅) (CH₃) C (OH) COOH
5. 2-(4'-Hydroxyphenyl) 2-hydroxyethanoic acid (4-Hydroxymandelic acid)
   (HO-C₆H₄) (H) C (OH) COOH
6. 2-(4'-Chlorophenyl) 2-hydroxyethanoic acid (4-Chloromandelic acid)
   (Cl-C₆H₄) (H) C (OH) COOH
7. 2-(3'-Hydroxy-4'-methoxyphenyl) 2-hydroxyethanoic acid (3-Hydroxy-4-methoxymandelic acid)
   (HO-,CH₃O-C₆H₃) (H) C (OH) COOH
8. 2-(4'-Hydroxy-3'-methoxyphenyl) 2-hydroxyethanoic acid (4-Hydroxy-3-methoxymandelic acid)
   (HO-,CH₃O-C₆H₃) (H) C (OH) COOH
9. 3-(2'-Hydroxyphenyl) 2-hydroxypropanoic acid [3-(2'Hydroxyphenyl) lactic acid]
   (HO-C₆H₄-CH₂) (H) C (OH) COOH
10. 3-(4'-Hydroxyphenyl) 2-hydroxypropanoic acid [3-(4'-Hydroxyphenyl) lactic acid]
   (HO-C₆H₄-CH₂) (H) C (OH) COOH
11. 2-(3',4'-Dihydroxyphenyl) 2-hydroxyethanoic acid (3,4-Dihydroxymandelic acid)
   (HO-,HO-C₆H₃) (H) C (OH) COOH

### (3) Polyhydroxy-carboxylic Acids

1. 2,3-Dihydroxypropanoic acid (Glyceric acid)
   (HOCH₂) (H) C (OH) COOH
2. 2,3,4-Trihydroxybutanoic acid (Isomers; erythronic acid, threonic acid)
   (HOCH₂ HOCH) (H) C (OH) COOH
3. 2,3,4,5-Tetrahydroxypentanoic acid (Isomers; ribonic acid, arabinoic acid, xylonic acid, lyxonic acid)
   (HOCH₂ HOCH HOCH) (H) C (OH) COOH
4. 2,3,4,5,6-Pentahydroxyhexanoic acid (Isomers; allonic acid, altronic acid, gluconic acid, mannoic acid, gulonic acid, idonic acid, galactonic acid, talonic acid)
   (HOCH₂ HOCH HOCH HOCH) (H) C (OH) COOH
5. 2,3,4,5,6,7-Hexahydroxyheptanoic acid (Isomers; glucoheptonic acid, galactoheptonic acid etc.)
   (HOCH₂ HOCH HOCH HOCH HOCH) (H) C (OH) COOH

### (4) Hydroxy-polycarboxylic Acids

1. 2-Hydroxypropane-1,3-dioic acid (Tartronic acid)
   (HOOC) (H) C (OH) COOH
2. 2-Hydroxybutane-1,4-dioic acid (Malic acid)
   (HOOC CH₂) (H) C (OH) COOH
3. 2,3-Dihydroxybutane-1,4-dioic acid (Tartaric acid)
   (HOOC HOCH) (H) C (OH) COOH
4. 2-Hydroxy-2-carboxypentane-1,5-dioic acid (Citric acid)
   (HOOC CH₂)₂ C (OH) COOH
5. 2,3,4,5-Tetrahydroxyhexane-1,6-dioic acid (Isomers; saccharic acid, mucic acid etc.)
   HOOC (CHOH)₄ COOH

The 2-hydroxycarboxylic acids may be present in forms other than the acid, such as, for example, salts or lactones. Typical lactone forms which may be used in accordance with this invention include, for example, gluconolactone, galactonolactone, glucuronolactone, galacturonolactone, gulonolactone, ribonolactone, saccharic acid lactone, pantoyllactone, glucoheptonolactone, mannonolactone, and galactoheptonolactone.

### Group 2

The second group, which comprises compounds related to the 2-hydroxycarboxylic acids, includes organic carboxylic acids in which one keto group is attached to position 2 carbon atom of the acid. The generic structure of such 2-ketoacids may be represented as follows:
(R_{c}) CO COO (R_{d})
wherein R_{c} and R_{d} can be the same or different and are each selected from H, alkyl, aralkyl or aryl group of saturated or unsaturated, isomeric or non-isomeric, straight or branched chain or cyclic form, having 1 to 29 carbon atoms, and in addition R_{c} may carry F, Cl, Br, I, OH, CHO, COOH and alkoxy group having 1 to 9 carbon atoms. The alpha ketoacids may be present as a free acid or an ester form, or in a salt form with an organic base or an inorganic alkali. The typical alkyl, aralkyl and aryl groups for R_{c} and R_{d} include methyl, ethyl, propyl, 2-propyl, butyl, pentyl, hexyl, octyl, dodecyl, hexadecyl, benzyl and phenyl.

The representative 2-ketocarboxylic acids and their esters of the second group are listed below:
1. 2-Ketoethanoic acid (Glyoxylic acid)
   (H) CO COOH
2. Methyl 2-ketoethanoate
   (H) CO COOCH₃
3. 2-Ketopropanoic acid (Pyruvic acid)
   CH₃ CO COOH
4. Methyl 2-ketopropanoate (Methyl pyruvate)
   CH₃ CO COOCH₃
5. Ethyl 2-ketopropanoate (Ethyl pyruvate)
   CH₃ CO COOC₂H₅
6. Propyl 2-ketopropanoate (Propyl pyruvate)
   CH₃ CO COOC₃H₇
7. 2-Phenyl-2-ketoethanoic acid (Benzoylformic acid)
   C₆H₅ CO COOH
8. Methyl 2-phenyl-2-ketoethanoate (Methyl benzoylformate)
   C₆H₅ CO COOCH₃
9. Ethyl 2-phenyl-2-ketoethanoate (Ethyl benzoylformate)
   C₆H₅ CO COOC₂H₅
10. 3-Phenyl-2-ketopropanoic acid (Phenylpyruvic acid)
   C₆H₅CH₂ CO COOH
11. Methyl 3-phenyl-2-ketopropanoate (Methyl phenylpyruvate)
   C₆H₅CH₂ CO COOCH₃
12. Ethyl 3-phenyl-2-ketopropanoate (Ethyl phenylpyruvate)
   C₆H₅CH₂ CO COOC₂H₅
13. 2-ketobutanoic acid
   C₂H₅ CO COOH
14. 2-Ketopentanoic acid
   C₃H₇ CO COOH
15. 2-Ketohexanoic acid
   C₄H₉ CO COOH
16. 2-Ketoheptanoic acid
   C₅H₁₁ CO COOH
17. 2-Ketooctanoic acid
   C₆H₁₃ CO COOH
18. 2-Ketododecanoic acid
   C₁₀H₂₁ CO COOH
19. Methyl 2-ketooctanoate
   C₆H₁₄ CO COOCH₃

### Group 3

The third group, which also comprises related compounds, includes, *inter alia*, hydroxycarboxylic acids where the hydroxy is at a position other than position 2, and cyclic hydroxycarboxylic acids. The members of this group, which are more conveniently identified by name than by generic structures, include ascorbic acid, quinic acid, isocitric acid, tropic acid (2-phenyl 3-hydroxypropanoic acid), trethocanic acid, 3-chlorolactic acid, citramalic acid, agaricic acid, aleuritic acid, pantoic acid, lactobionic acid and hexulosonic acid.

### Specific Compositions

While 2-hydroxycarboxylic acids and related compounds are therapeutically effective for topical treatment to improve or alleviate signs of nail changes associated with intrinsic aging and/or photoaging, certain compounds of the instant invention are more potent than others. In selecting a particular compound of the present invention two factors, namely (a) potency and (b) concentration have to be considered. If rapid results are preferred in certain cases, most potent compounds with highest and safe concentrations may be used. Under such conditions the treatment time is substantially shortened with good to excellent clinical results. Generally, such treatment has to be carried out under supervision by a dermatologist or trained professional in the office, medical center, skin care center, or beauty salon etc. Such procedure or treatment may include micro and semimicro peels, epidermolysis or superficial peel, and dermolysis or deeper peel.

Examples of more potent 2-hydroxycarboxylic acids and related compounds to be formulated in specific compositions include 2-hydroxyethanoic acid, 2-hydroxypropanoic acid, 2-methyl 2-hydroxypropanoic acid, 2-phenyl 2-hydroxyethanoic acid, 2,2-diphenyl 2-hydroxyethanoic acid, 2-phenyl 2-methyl 2-hydroxyethanoic acid, 2-phenyl 3-hydroxypropanoic acid, 2-ketopropanoic acid, methyl 2-ketopropanoate and ethyl 2-ketopropanoate. The concentration of 2-hydroxycarboxylic acid or the related compound used in such specific composition may range from an intermediate to a full strength, therefore the dispensing and the application require special handling and procedures.

If the 2-hydroxycarboxylic acid or the related compound at full strength (usually 85-100%) is a liquid form at room temperature such as 2-hydroxypropanoic acid, 2-ketopropanoic acid, methyl 2-ketopropanoate and ethyl 2-ketopropanoate, the liquid compound with or without a gelling agent is directly dispensed as 0.5 to 1 ml aliquots in small vials.

If the 2-hydroxycarboxylic acid or the related compound at full strength is a crystalline or solid form at room temperature such as 2-hydroxyethanoic acid, 2-methyl 2-hydroxypropanoic acid, 2-phenyl 2-hydroxyethanoic acid, 2,2-diphenyl 2-hydroxyethanoic acid and 2-phenyl 3-hydroxypropanoic acid, the crystalline or solid compound is first dissolved in a minimal amount of vehicle or vehicle system prepared from water, ethanol, propylene glycol and/or butylene glycol with or without a gelling agent. For example, 2-hydroxyethanoic acid 70 g is dissolved in water 30 ml, and the 70% strength solution thus obtained is dispensed as 0.5 to 1 ml aliquots in small vials. If a gelling agent is used 0.1 to 2% of hydroxyethyl cellulose, methyl cellulose, hydroxypropyl cellulose, chitosan, carbomer, or polyquaternium-10 may be incorporated into the above solution.

To formulate an intermediate strength (usually 20-50%), 2-hydroxycarboxylic acid or the related compound either a liquid or solid form at room temperature is first dissolved in a vehicle or vehicle system prepared from water, acetone, ethanol, propylene glycol and/or butylene glycol. For example, 2-hydroxyethanoic acid or 2-ketopropanoic acid 30 g is dissolved in ethanol 56 g and propylene glycol 14 g, and the 30% strength solution thus obtained is dispensed as 7 to 14 ml aliquots in dropper bottles.

### General Preparation of Compositions

Most compositions of the instant invention may be formulated as solution, gel, lotion, cream, ointment, or other pharmaceutically acceptable form. To prepare a composition in solution form for general use, at least one 2-hydroxycarboxylic acid or related compound is dissolved in a solution prepared from ethanol, water, propylene glycol, butylene glycol, acetone or other pharmaceutically acceptable vehicle. The concentration of the 2-hydroxycarboxylic acid or related compound may range from 0.1 to 100 percent, the preferred concentration ranges being from about 2 to about 25 percent for home use, with higher ranges, e.g., from about 70 to about 100 percent being acceptable for office use where professional supervision is provided. Thus, such concentrations can also range from about 25 to about 50 percent and from about 50 to about 70 percent, with the proviso that concentrations of about 25 percent or more generally requiring profession supervision.

In the preparation of a composition in lotion, cream or ointment form, at least one of 2-hydroxycarboxylic acids or related compounds is initially dissolved in a solvent such as water, ethanol, butylene glycol, and/or propylene glycol. The solution thus prepared is then mixed in a conventional manner with commonly available cream or ointment base such as hydrophilic ointment or petrolatum. The concentrations of 2-hydroxycarboxylic acids or related compounds used in the compositions are the same as described above.

A typical gel composition of the instant invention is formulated by dissolving at least one of 2-hydroxycarboxylic acids or related compounds in a vehicle prepared from ethanol, water, butylene glycol, and/or propylene glycol. A gelling agent such as xanthan gum, polyquaternium-10, methyl cellulose, ethyl cellulose, hydroxyethylcellulose, hydroxypropylcellulose, chitosan, hydroxypropylmethylcellulose, ammoniated glycyrrhizinate or carbomer is then added to the solution with agitation. The preferred concentration of the gelling agent may range from 0.1 to 2 percent by weight of the total composition.

The following are illustrative examples of formulations and compositions according to this invention. Although the examples utilize only selected compounds and formulations, it should be understood that the following examples are illustrative and not limited. Therefore, any of the aforementioned 2-hydroxycarboxylic acids and related compounds may be substituted according to the teachings of this invention in the following examples.

### EXAMPLE 1

A typical solution composition containing 2-hydroxycarboxylic acid or the related compound may be formulated as follows. 2-Hydroxyethanoic acid (glycolic acid) crystals 7 g is dissolved in water 50 ml and propylene glycol 15 ml. Ethanol is added to the solution until the total volume is 100 ml. The composition thus prepared contains 7% w/v 2-hydroxyethanoic acid.

### EXAMPLE 2

A typical gel composition containing 2-hydroxycarboxylic acid or the related compound may be formulated as follows.

2-Hydroxypropanoic acid (DL-lactic acid) USP grade 5 g is dissolved in water 60 ml and butylene glycol 10 ml, and chitosan or polyquaternium-10 0.3 g is added with stirring. Ethanol is added to the mixture until the volume is 100 ml. The mixture is stirred until a uniform gel is obtained. The thin gel thus obtained contains 5% 2-hydroxypropanoic acid.

### EXAMPLE 3

A typical oil-in-water emulsion containing 2-hydroxycarboxylic acid or the related compound may be formulated as follows.

2-Methyl 2-hydroxypropanoic acid (methyllactic acid) crystals 10 g is dissolved in water 20 ml and concentrated ammonium hydroxide 2 ml is added to the solution. The solution is mixed with enough hydrophilic ointment USP to make a total weight of 100 g. The cream thus formulated contains 10% 2-methyl 2-hydroxypropanoic acid.

### EXAMPLE 4

A typical water-in-oil emulsion containing 2-hydroxycarboxylic acid or the related compound may be formulated as follows.

Gluconolactone 7 g is dissolved in water 12 ml and concentrated ammonium hydroxide 0.5 ml is added to the solution. The solution is mixed with enough water-in-oil emulsion to make a total weight of 100 g. The water non-washable cream thus formulated contains 7% gluconolactone.

### EXAMPLE 5

A typical ointment containing 2-hydroxycarboxylic acid or the related compound may be formulated as follows.

2-Phenyl 2-hydroxyethanoic acid (mandelic acid) crystals 10 g is dissolved in 10 ml ethanol, and the solution thus formed is mixed with mineral oil 35 g and enough white petrolatum to make a total weight of 100 g. The ointment thus formulated contains 10% 2-phenyl 2-hydroxyethanoic acid.

### EXAMPLE 6

A specific preparation containing a full strength or a high concentration of 2-hydroxycarboxylic acid or the related compound may be formulated and dispensed as follows.

If 2-hydroxycarboxylic acid or the related compound at full strength is a liquid form at room temperature such as 2-hydroxypropanoic acid, 2-ketopropanoic acid, methyl 2-ketopropanoate and ethyl 2-ketopropanoate, the compound is directly dispensed as 0.5 to 1 ml aliquots in small vials. If the compound is a crystalline or solid form, such as 2-hydroxyethanoic acid, 2-methyl 2-hydroxypropanoic acid, 2-phenyl 2-hydroxyethanoic acid, 2-phenyl 3-hydroxypropanoic acid, 2-phenyl 2-methyl 2-hydroxyethanoic acid and 2,2-diphenyl 2-hydroxyethanoic acid, the compound is first dissolved in minimal amount of an appropriate vehicle system selected from water, ethanol, propylene glycol and butylene glycol with or without a gelling agent. For example, 2-hydroxyethanoic acid 70 g is dissolved in water 30 ml, and 70% strength 2-hydroxyethanoic acid with or without addition of 0.5% chitosan or polyquaternium-10 is dispensed as 1 to 5 ml aliquots in small vials.

### EXAMPLE 7

A typical preparation containing an intermediate strength of 2-hydroxycarboxylic acid or the related compound may be formulated as follows.

Malic acid, tartaric acid or citric acid 35 g is dissolved in water 60 ml and propylene glycol 5 ml. The 35% strength solution thus prepared is dispensed as 5 to 10 ml aliquots in dropper bottles.

### TEST RESULTS

### (1) Biologic and Pharmacologic Actions

The skin may be classified into two major parts; dermis and epidermis. The dermis contains blood vessels, nerves, collagen, elastin etc, and fibroblast cells in the dermis are responsible for the biosynthesis of collagen and elastin. The epidermis contains nerves but no collagen, elastin, nor blood vessels.

The epidermis is further divided into two distinct zones; malpighian layer and horny layer. The malpighian layer, a living tissue, is further divided into basal, spinous, and granular layers. The horny layer, a dead tissue, is also called stratum corneum. In the natural process, basal cells in the basal layer move outward through the spinous and granular layers to become dead cells called corneocytes, in the stratum corneum. The stratum corneum consists of approximately 14 layers of corneocytes. In normal skin it takes about 14 days for the basal cells to move from the basal layer to the end of the granular layer and to become corneocytes, and another 14 days to reach the outermost layer of the stratum corneum. This process of forming corneocytes is called keratinization, and stratum corneum, nail, and hair are the natural products produced by such process. The stratum corneum is the skin tissue that one feels when touching the skin. Usually, it takes about 28 days for cells of the basal layer to move outward to the surface in the course of making new skin.

We have found that compositions containing low concentrations of 2-hydroxycarboxylic acid or the related compound, when applied topically to the skin, diminish corneocyte cohesion in the stratum corneum. This effect predominantly occurs among corneocyte cells at inner levels of the stratum corneum, i.e. near the junction to the granular layer, and there is no effect among corneocyte cells at outer layers in the stratum corneum. Therefore, 2-hydroxycarboxylic acids and related compounds are not typical keratolytics such as strong acids, strong alkalis, thiols, urea and lithium salts which cause disaggregation of corneocyte cells in the outer layers of the stratum corneum.

We have also discovered that compositions containing intermediate to high concentrations of 2-hydroxycarboxylic acid or the related compound, when topically applied to the skin, cause profound beneficial effects in the dermis as well as the epidermis of the skin. The skin becomes thicker and plump as measured clinically by caliper and micrometer techniques. Histometric techniques using microscopic analysis of tissue biopsy specimens confirm that new and more collagen and elastic fibers have been biosynthesized in the dermis.

The biologic and pharmacologic actions of 2-hydroxycarboxylic acid or the related compound suggest that topical application of the composition should improve or alleviate signs of nail changes associated with intrinsic and/or extrinsic aging.

### (2) Nail Treatments

Compositions containing 2-hydroxycarboxylic acid or the related compound at intermediate concentrations, preferably from 8 to 20%, for nail care and treatment were formulated according to the examples. A solution or thin gel form thus prepared was topically applied twice daily to edges, surface and base of affected nail plates. After a few months of such treatment, the signs of nail changes associated with intrinsic and extrinsic aging started to improve noticeably. The nail looked glossy and felt smooth on the surface. The flexibility and elasticity of the nail after the treatment also increased. Brittleness diminished and the occurrence of terminal nail splitting became rare.

It will be apparent to those skilled in the art that various modifications and variations can be made to the compositions of matter and methods of this invention. Thus, it is intended that the present invention covers such modifications and variations.

## Claims

1. Use of a cosmetic composition comprising one or more 2-hydroxycarboxylic acids, 2-ketoacids, or related compounds, said related compounds selected from the group consisting of ascorbic acid, quinic acid, isocitric acid, tropic acid, trethocanic acid, 3-chlorolactic acid, cerebronic acid, citramalic acid, agaricic acid, aleuritic acid, pantoic acid, lactobionic acid and hexulosonic acid, in a topically acceptable vehicle for topical application to the nail of humans or animals for alleviating signs of nail changes associated with intrinsic and/or extrinsic aging.

2. Use of a composition as claimed in claim 1, wherein said extrinsic aging include any or plural of sunlight; radiations; air pollution; wind; cold; dampness; heat; chemicals; smoke; cigarette smoking.

3. Use of a composition as claimed in any of claims 1 or 2, wherein the signs of nail changes associated with intrinsic and/or extrinsic aging include thinning, fragility, splitting, lack of luster, uneven surface, loss of flexibility, loss of elasticity, and loss of resiliency.

4. Use of a composition as claimed in any of claims 1-3, wherein said 2-hydroxycarboxylic acid may be represented by a generic structure of:
(Rₐ) (R_{b}) C (OH) COOH
wherein Rₐ and R_{b} may be the same or different and are independently selected from H, F, Cl, Br, alkyl, aralkyl or aryl group of saturated or unsaturated, isomeric or non-isomeric, straight or branched chain or cyclic form, having 1 to 29 carbon atoms, and in addition Rₐ and R_{b} may carry OH, CHO, COOH and alkoxy group having 1 to 9 carbon atoms, said 2-hydroxycarboxylic acid may be present as a free acid or lactone form, or in a salt form with an organic base or an inorganic alkali, and as stereoisomers as D, L, and DL forms when Rₐ and R_{b} are not identical.

5. Use of a composition as claimed in claim 4, wherein said 2-hydroxycarboxylic acid is in salt or lactone form, preferably selected from the group consisting of gluconolactone, galactonolactone, glucuronolactone, galactrononolactone, gulonolactone, ribonolactone, saccharic acid lactone, pantoyllactone, glucoheptonolactone, mannonolactone, and galactoheptonolactone.

6. Use of a composition as claimed in claim 4, wherein said 2-hydroxycarboxylic acid is an alkyl hydroxycarboxylic acid, preferably selected from the group consisting of 2-Hydroxyethanoic acid (Glycolic acid), 2-Hydroxypropanoic acid (Lactic acid), 2-Methyl 2-hydroxypropanoic acid (Methyllactic acid), 2-Hydroxybutanoic acid, 2-Hydroxypentanoic acid, 2-Hydroxyhexanoic acid, 2-Hydroxyheptanoic acid, 2-Hydroxyoctanoic acid, 2-Hydroxynonanoic acid, 2-Hydroxydecanoic acid, 2-Hydroxyundecanoic acid, 2-Hydroxydodecanoic acid (Alpha hydroxylauric acid), 2-Hydroxytetradecanoic acid (Alpha hydroxymyristic acid), 2-Hydroxyhexadecanoic acid (Alpha hydroxypalmitic acid), 2-Hydroxyoctadecanoic acid (Alpha hydroxystearic acid), 2-Hydroxyeicosanoic acid (Alpha hydroxyarachidonic acid), 2-Hydroxytetraeicosanoic acid (Cerebronic acid), and 2-Hydroxytetraeicosenoic acid (Alpha hydroxynervonic acid).

7. Use of a composition as claimed in claim 4, wherein said 2-hydroxycarboxylic acid is an aralkyl or aryl 2-hydroxycarboxylic acid, preferably selected from the group consisting of 2-Phenyl 2-hydroxyethanoic acid (Mandelic acid), 2,2-Diphenyl 2-hydroxyethanoic acid (Benzilic acid), 3-Phenyl 2-hydroxypropanoic acid (Phenyllactic acid), 2-Phenyl 2-methyl 2-hydroxyethanoic acid (Atrolactic acid), 2-(4'-Hydroxyphenyl) 2-hydroxyethanoic acid, 2-(4'-Chlorophenyl) 2-hydroxyethanoic acid, 2-(3'-Hydroxy-4'-methoxyphenyl) 2-hydroxyethanoic acid, 2-(4'-Hydroxy-3'-methoxyphenyl) 2-hydroxyethanoic acid, 3-(2'-Hydroxyphenyl) 2-hydroxypropanoic acid, 3-(4'-Hydroxyphenyl) 2-hydroxypropanoic acid, and 2-(3',4'-Dihydroxyphenyl) 2-hydroxyethanoic acid.

8. Use of a composition as claimed in claim 4, wherein said 2-hydroxycarboxylic acid is a polyhydroxycarboxylic acid or hydroxypolycarboxylic acid, preferably selected from the group consisting of 2,3-Dihydroxypropanoic acid (Glyceric acid), 2,3,4-Trihydroxybutanoic acid (Isomers; erythronic acid, threonic acid), 2,3,4,5-Tetrahydroxypentanoic acid (Isomers; ribonic acid, arabinoic acid, xylonic acid, lyxonic acid), 2,3,4,5,6-Pentahydroxyhexanoic acid (Isomers; allonic acid, altronic acid, gluconic acid, mannoic acid, gulonic acid, idonic acid, galactonic acid, talonic acid), 2,3,4,5,6,7-Hexahydroxyheptanoic acid (Isomers; glucoheptonic acid, galactoheptonic acid etc.) and Hydroxypropane-1,3-dioic acid (Tartronic acid), 2-Hydroxybutane-1,4-dioic acid (Malic acid), 2,3-Dihydroxybutane-1,4-dioic acid (Tartaric acid), 2-Hydroxy-2-carboxypentane-1,5-dioic acid (Citric acid), 2,3,4,5-Tetrahydroxyhexane-1,6-dioic acid (Isomers; saccharic acid, mucic acid, etc.).

9. Use of a composition as claimed in any of claims 1-3, wherein said related compound comprises a 2-ketoacid of the generic formula:
(R_{c}) CO COO (R_{d})
wherein R_{c} and R_{d} may be the same or different and are independently selected from H, alkyl, aralkyl or aryl group of saturated or unsaturated, isomeric or non-isomeric, straight or branched chain or cyclic form, having 1 to 29 carbon atoms, and in addition R_{c} may carry F, Cl, Br, I, OH, CHO, COOH and alkoxy group having 1 to 9 carbon atoms, said alpha ketoacid existing as a free acid or an ester form, or in a salt form with an organic base or an inorganic alkali.

10. Use of a composition as claimed in claim 9, wherein said 2-ketoacid is selected from the group consisting of 2-Ketoethanoic acid (Glyoxylic acid), Methyl 2-ketoethanoate, 2-Ketopropanoic acid (Pyruvic acid), Methyl 2-ketopropanoate (Methyl pyruvate), Ethyl 2-ketopropanoate (Ethyl pyruvate), Propyl 2-ketopropanoate (Propyl pyruvate), 2-Phenyl-2-ketoethanoic acid (Benzoylformic acid), Methyl 2-phenyl-2-ketoethanoate (Methyl benzoylformate), Ethyl 2-phenyl-2-ketoethanoate (Ethyl benzoylformate), 3-Phenyl-2-ketopropanoic acid (Phenylpyruvic acid), Methyl 3-phenyl-2-keotpropanoate (Methyl phenylpyruvate), Ethyl 3-phenyl-2-ketopropanoate (Ethyl phenylpyruvate), 2-Ketobutanoic acid, 2-Ketopentanoic acid, 2-Ketohexanoic acid, 2-Ketoheptanoic acid, 2-Ketooctanoic acid, 2-Ketododecanoic acid, and Methyl 2-ketooctanoate.

11. Use of a composition as claimed in claim 10 wherein said related compound is a 2-ketocarboxylic acid ester.

12. Use of a composition as claimed in any of claims 1-3, wherein said 2-hydroxycarboxylic acid or related compound is selected from the group consisting of isocitric acid, methyllactic acid, mandelic acid, quinic acid or quinolactone, malic acid, ethyl pyruvate, tropic acid, ascorbic acid or ascorbolactone, benzilic acid, ribonic acid or ribonolactone, saccharic acid or saccharolactone, mucic acid or mucolactone, citramalic acid, galactonic acid or galactonolactone, gulonic acid or gulonolactone, glucoheptonic acid or glucoheptonolactone, pantoic acid or pantolactone, phenyllactic acid, gluconic acid or gluconolactone.

13. Use of one or more 2-hydroxycarboxylic acids, 2-ketoacids, or related compounds, said related compunds selected from the group consisting of ascorbic acid, quinic acid, isocitric acid, tropic acid, trethocanic acid, 3-chlorolactic acid, cerebronic acid, citramalic acid, agaricic acid, aleuritic acid, pantoic acid, lactobionic acid and hexulosonic acid, together with a vehicle acceptable for topical application to the nail of humans or animals, for the preparation of a therapeutic composition for alleviating signs of nail changes associated with intrinsic and/or extrinsic aging.

14. Use as claimed in claim 13, wherein said extrinsic aging include any or plural of sunlight; radiations; air pollution; wind; cold; dampness; heat; chemicals; smoke; cigarette smoking.

15. Use as claimed in any of claims 13 or 14, wherein the signs of nail changes associated with intrinsic and/or extrinsic aging include thinning, fragility, splitting, lack of luster, uneven surface, loss of flexibility, loss of elasticity, and loss of resiliency.

16. Use as claimed in any of claims 13-15, wherein said 2-hydroxycarboxylic acid may be represented by a generic structure of:
(Rₐ) (R_{b}) C (OH) COOH
wherein Rₐ and R_{b} may be the same or different and are independently selected from H, F, Cl, Br, alkyl, aralkyl or aryl group of saturated or unsaturated, isomeric or non-isomeric, straight or branched chain or cyclic form, having 1 to 29 carbon atoms, and in addition Rₐ and R_{b} may carry OH, CHO, COOH and alkoxy group having 1 to 9 carbon atoms, said 2-hydroxycarboxylic acid may be present as a free acid or lactone form, or in a salt form with an organic base or an inorganic alkali, and as stereoisomers as D, L, and DL forms when Rₐ and R_{b} are not identical.

17. Use as claimed in claim 16, wherein said 2-hydroxycarboxylic acid is in salt or lactone form, preferably selected from the group consisting of gluconolactone, galactonolactone, glucuronolactone, galactrononolactone, gulonolactone, ribonolactone, saccharic acid lactone, pantoyllactone, glucoheptonolactone, mannonolactone, and galactoheptonolactone.

18. Use as claimed in claim 16, wherein said 2-hydroxycarboxylic acid is an alkyl hydroxycarboxylic acid, preferably selected from the group consisting of 2-Hydroxyethanoic acid (Glycolic acid), 2-Hydroxypropanoic acid (Lactic acid), 2-Methyl 2-hydroxypropanoic acid (Methyllactic acid), 2-Hydroxybutanoic acid, 2-Hydroxypentanoic acid, 2-Hydroxyhexanoic acid, 2-Hydroxyheptanoic acid, 2-Hydroxyoctanoic acid, 2-Hydroxynonanoic acid, 2-Hydroxydecanoic acid, 2-Hydroxyundecanoic acid, 2-Hydroxydodecanoic acid (Alpha hydroxylauric acid), 2-Hydroxytetradecanoic acid (Alpha hydroxymyristic acid), 2-Hydroxyhexadecanoic acid (Alpha hydroxypalmitic acid), 2-Hydroxyoctadecanoic acid (Alpha hydroxystearic acid), 2-Hydroxyeicosanoic acid (Alpha hydroxyarachidonic acid), 2-Hydroxytetraeicosanoic acid (Cerebronic acid), and 2-Hydroxytetraeicosenoic acid (Alpha hydroxynervonic acid).

19. Use as claimed in claim 16, wherein said 2-hydroxycarboxylic acid is an aralkyl or aryl 2-hydroxycarboxylic acid, preferably selected from the group consisting of 2-Phenyl 2-hydroxyethanoic acid (Mandelic acid), 2,2-Diphenyl 2-hydroxyethanoic acid (Benzilic acid), 3-Phenyl 2-hydroxypropanoic acid (Phenyllactic acid), 2-Phenyl 2-methyl 2-hydroxyethanoic acid (Atrolactic acid), 2-(4'-Hydroxyphenyl) 2-hydroxyethanoic acid, 2-(4'-Chlorophenyl) 2-hydroxyethanoic acid, 2-(3'-Hydroxy-4'-methoxyphenyl) 2-hydroxyethanoic acid, 2(4'-Hydroxy-3'-methoxyphenyl) 2-hydroxyethanoic acid, 3-(2'-Hydroxyphenyl) 2-hydroxypropanoic acid, 3-(4'-Hydroxyphenyl) 2-hydroxypropanoic acid, and 2-(3',4'-Dihydroxyphenyl) 2-hydroxyethanoic acid.

20. Use as claimed in claim 16, wherein said 2-hydroxycarboxylic acid is a polyhydroxycarboxylic acid or hydroxypolycarboxylic acid, preferably selected from the group consisting of 2,3-Dihydroxypropanoic acid (Glyceric acid), 2,3,4-Trihydroxybutanoic acid (Isomers; erythronic acid, threonic acid), 2,3,4,5-Tetrahydroxypentanoic acid (Isomers; ribonic acid, arabinoic acid, xylonic acid, lyxonic acid), 2,3,4,5,6-Pentahydroxyhexanoic acid (Isomers; allonic acid, altronic acid, gluconic acid, mannoic acid, gulonic acid, idonic acid, galactonic acid, talonic acid), 2,3,4,5,6,7-Hexahydroxyheptanoic acid (Isomers; glucoheptonic acid, galactoheptonic acid etc.) and Hydroxypropane-1,3-dioic acid (Tartronic acid), 2-Hydroxybutane-1,4-dioic acid (Malic acid), 2,3-Dihydroxybutane-1,4-dioic acid (Tartaric acid), 2-Hydroxy-2-carboxypentane-1,5-dioic acid (Citric acid), 2,3,4,5-Tetrahydroxyhexane-1,6-dioic acid (Isomers; saccharic acid, mucic acid, etc.).

21. Use as claimed in any of claims 13-15, wherein said related compound comprises a 2-ketoacid of the generic formula:
(R_{c}) CO COO (R_{d})
wherein R_{c} and R_{d} may be the same or different and are independently selected from H, alkyl, aralkyl or aryl group of saturated or unsaturated, isomeric or non-isomeric, straight or branched chain or cyclic form, having 1 to 29 carbon atoms, and in addition R_{c} may carry F, Cl, Br, I, OH, CHO, COOH and alkoxy group having 1 to 9 carbon atoms, said alpha ketoacid existing as a free acid or an ester form, or in a salt form with an organic base or an inorganic alkali.

22. Use as claimed in claim 21, wherein said 2-ketoacid is selected from the group consisting of 2-Ketoethanoic acid (Glyoxylic acid), Methyl 2-ketoethanoate, 2-Ketopropanoic acid (Pyruvic acid), Methyl 2-ketopropanoate (Methyl pyruvate), Ethyl 2-ketopropanoate (Ethyl pyruvate), Propyl 2-ketopropanoate (Propyl pyruvate), 2-Phenyl-2-ketoethanoic acid (Benzoylformic acid), Methyl 2-phenyl-2-ketoethanoate (Methyl benzoylformate), Ethyl 2-phenyl-2-ketoethanoate (Ethyl benzoylformate), 3-Phenyl-2-ketopropanoic acid (Phenylpyruvic acid), Methyl 3-phenyl-2-keotpropanoate (Methyl phenylpyruvate), Ethyl 3-phenyl-2-ketopropanoate (Ethyl phenylpyruvate), 2-Ketobutanoic acid, 2-Ketopentanoic acid, 2-Ketohexanoic acid, 2-Ketoheptanoic acid, 2-Ketooctanoic acid, 2-Ketododecanoic acid, and Methyl 2-ketooctanoate.

23. Use as claimed in claim 22 wherein said related compound is a 2-ketocarboxylic acid ester.

24. Use as claimed in any of claims 13-15, wherein said 2-hydroxycarboxylic acid or related compound is selected from the group consisting of isocitric acid, methyllactic acid, mandelic acid, quinic acid or quinolactone, malic acid, ethyl pyruvate, tropic acid, ascorbic acid or ascorbolactone, benzilic acid, ribonic acid or ribonolactone, saccharic acid or saccharolactone, mucic acid or mucolactone, citramalic acid, galactonic acid or galactonolactone, gulonic acid or gulonolactone, glucoheptonic acid or glucoheptonolactone, pantoic acid or pantolactone, phenyllactic acid, gluconic acid or gluconolactone.

25. Use of a composition as claimed in any of claims 1 to 4, wherein said 2-hydroxy-carboxylic acid, 2-ketoacid, or related compounds, is 2-hydroxyethanoic acid (glycolic acid).

26. Use of a composition as claimed in any of claims 1 to 4, wherein said 2-hydroxy-carboxylic acid, 2-ketoacid, or related compounds, is 2-hydroxypropanoic acid (lactic acid).

27. Use of a composition as claimed in any of claims 1 to 4, wherein said 2-hydroxy-carboxylic acid, 2-ketoacid, or related compounds, is 2-hydroxy-2-carboxypentane-1,5-dioic acid (citric acid).

28. Use of a composition as claimed in any of claims 1 to 4, wherein said 2-hydroxy-carboxylic acid, 2-ketoacid, or related compounds, is 2-methyl 2-hydroxypropanoic acid (methyllactic acid).

29. Use of a composition as claimed in any of claims 1 to 4, wherein said 2-hydroxy-carboxylic acid, 2-ketoacid, or related compounds, is gluconolactone.

30. Use of a composition as claimed in any of claims 1 to 4, wherein said 2-hydroxy-carboxylic acid, 2-ketoacid, or related compounds, is quinic acid, ribonolactone, malic acid, mandelic acid, benzilic acid, galactonolactone, ascorbic acid, ethyl pyruvate or tropic acid.

31. Use as claimed in any of claims 13-17, wherein said 2-hydroxycarboxylic acids, 2-ketoacids, or related compounds, is 2-hydroxyethanoic acid (glycolic acid).

32. Use as claimed in any of claims 13-17, wherein said 2-hydroxycarboxylic acid, 2-ketoacid, or related compounds, is 2-hydroxypropanoic acid (lactic acid).

33. Use as claimed in any of claims 13-17, wherein said 2-hydroxycarboxylic acid, 2-ketoacid, or related compounds, is 2-hydroxy-2-carboxypentane-1,5-dioic acid (citric acid).

34. Use as claimed in any of claims 13-17, wherein said 2-hydroxycarboxylic acid, 2-ketoacid, or related compounds, is 2-methyl 2-hydroxypropanoic acid (methyllactic acid).

35. Use as claimed in any of claims 13-17, wherein said 2-hydroxycarboxylic acid, 2-ketoacid, or related compounds, is gluconolactone.

36. Use as claimed in any of claims 13-17, wherein said 2-hydroxycarboxylic acid, 2-ketoacid, or related compounds, is quinic acid, ribonolactone, malic acid, mandelic acid, benzilic acid, galactonolactone, ascorbic acid, ethyl pyruvate and tropic acid.

## Patentansprüche

1. Verwendung einer kosmetischen Zusammensetzung, enthaltend eine oder mehrere 2-Hydroxycarbonsäuren, 2-Ketosäuren oder verwandte Verbindungen, wobei die verwandten Verbindungen ausgewählt sind aus der aus Ascorbinsäure, Chinasäure, Isocitronensäure, Tropasäure, Trethocansäure, 3-Chlormilchsäure, Cerebronsäure, Citramalsäure, Agaricinsäure, Aleuritinsäure, Pantoinsäure, Lactobionsäure und Hexulosonsäure bestehenden Gruppe, in einem topisch annehmbaren Träger für die topische Aufbringung auf die Nägel von Menschen oder Tieren zur Linderung von Anzeichen von Nägelveränderungen, die mit intrinsischem und/oder extrinsischem Altern verbunden sind.

2. Verwendung einer Zusammensetzung nach Anspruch 1, worin das extrinsische Altern Sonnenlicht, Bestrahlungen, Luftverschmutzung, Wind, Kälte, Feuchtigkeit, Wärme oder Hitze, Chemikalien, Rauch, Zigarettenrauchen, und zwar eines oder mehrere unter den vorstehend genannten, einschließt.

3. Verwendung einer Zusammensetzung nach Anspruch 1 oder 2, worin die Anzeichen von Nägelveränderungen, die mit intrinsischem und/oder extrinsischem Altern verbunden sind, das Dünnerwerden, Brüchigwerden, Splittern, Glanzverlust, unebene Oberfläche, Biegsamkeitsverlust, Elastizitätsverlust und Verlust der Spannkraft umfassen.

4. Verwendung einer Zusammensetzung nach einem der Ansprüche 1 bis 3, worin die genannte 2-Hydroxycarbonsäure durch die allgemeine Struktur :
(Rₐ) (R_{b}) C (OH) COOH
dargestellt werden kann, worin Rₐ und R_{b} gleich oder verschieden sein können und unabhängig voneinander ausgewählt sind unter H, F, Cl, Br, Alkyl-, Aralkyl- or Arylgruppen in gesättigter oder ungesättigter, isomerer oder nicht isomerer, geradkettiger oder verzweigtkettiger oder cyclischer Form mit 1 bis 29 Kohlenstoffatomen, und weiterhin Rₐ und R_{b} eine OH-, CHO-, COOH- und Alkoxygruppe mit 1 bis 9 Kohlenstoffatomen tragen können, wobei die genannte 2-Hydroxycarbonsäure als freie Säure oder in Lactonform oder in Salzform mit einer organischen Base oder einem anorganischen Alkali vorliegen kann, sowie als Stereoisomere in D-, L- und DL-Form, wenn Rₐ und R_{b} nicht identisch sind.

5. Verwendung einer Zusammensetzung nach Anspruch 4, worin die genannte 2-Hydroxycarbonsäure in Salz- oder Lactonform vorliegt, vorzugsweise ausgewählt aus der aus Gluconolacton, Galactonolacton, Glucuronlacton, Galacturonlacton, Gulonlacton, Ribonolacton, Saccharinsäurelacton, Pantoyllacton, Glucoheptonlacton, Mannonlacton und Galactoheptonlacton bestehenden Gruppe.

6. Verwendung einer Zusammensetzung nach Anspruch 4, worin die genannte 2-Hydroxycarbonsäure eine Alkylhydroxycarbonsäure ist, vorzugsweise ausgewählt aus der aus 2-Hydroxyessigsäure (Glycolsäure), 2-Hydroxypropansäure (Milchsäure), 2-Methyl-2-hydroxypropansäure (Methylmilchsäure), 2-Hydroxybutansäure, 2-Hydroxypentansäure, 2-Hydroxyhexansäure, 2-Hydroxyheptansäure, 2-Hydroxyoctansäure, 2-Hydroxynonansäure, 2-Hydroxydecansäure, 2-Hydroxyundecansäure, 2-Hydroxydodecansäure (alpha-Hydroxylaurinsäure), 2-Hydroxytetradecansäure (alpha-Hydroxymyristinsäure), 2-Hydroxyhexadecansäure (alpha-Hydroxypalmitinsäure), 2-Hydroxyoctadecansäure (alpha-Hydroxystearinsäure), 2-Hydroxyeicosansäure (alpha-Hydroxyarachidonsäure), 2-Hydroxytetraeicosansäure (Cerebronsäure) und 2-Hydroxytetraeicosensäure (alpha-Hydroxynervonsäure) bestehenden Gruppe.

7. Verwendung einer Zusammensetzung nach Anspruch 4, worin die genannte 2-Hydroxycarbonsäure eine Aralkyl- or Aryl-2-hydroxycarbonsäure ist, vorzugsweise ausgewählt aus der aus 2-Phenyl-2-hydroxyessigsäure (Mandelsäure), 2,2-Diphenyl-2-hydroxyessigsäure (Benzilsäure), 3-Phenyl-2-hydroxypropansäure (Phenylmilchsäure), 2-Phenyl-2-methyl-2-hydroxyessigsäure (Atrolactinsäure), 2-(4'-Hydroxyphenyl)-2-hydroxyessigsäure, 2-(4'-Chlorophenyl)-2-hydroxyessigsäure, 2-(3'-Hydroxy-4'-methoxyphenyl)-2-hydroxyessigsäure, 2-(4'-Hydroxy-3^{'}-methoxyphenyl)-2-hydroxyessigsäure, 3-(2'-Hydroxyphenyl)-2-hydroxypropansäure, 3-(4'-Hydroxyphenyl)-2-hydroxypropansäure und 2-(3',4'-Dihydroxyphenyl)-2-hydroxyessigsäure bestehenden Gruppe.

8. Verwendung einer Zusammensetzung nach Anspruch 4, worin die genannte 2-Hydroxycarbonsäure eine Polyhydroxycarbonsäure oder Hydroxypolycarbonsäure ist, vorzugsweise ausgewählt aus der aus 2,3-Dihydroxypropansäure (Glycerinsäure), 2,3,4-Trihydroxybutansäure (Isomere; Erythronsäure, Threonsäure), 2,3,4,5-Tetrahydroxypentansäure (Isomere; Ribonsäure, Arabinsäure, Xylonsäure, Lyxonsäure), 2,3,4,5,6-Pentahydroxyhexansäure (Isomere; Allonsäure, Altronsäure, Gluconsäure, Mannonsäure, Gulonsäure, Idonsäure, Galactonsäure, Talonsäure), 2,3,4,5,6,7-Hexahydroxyheptansäure (Isomere; Glucoheptonsäure, Galactoheptonsäure etc.) und Hydroxypropan-1,3-disäure (Tartronsäure), 2-Hydroxybutan-1,4-disäure (Äpfelsäure), 2,3-Dihydroxybutan-1,4-disäure (Weinsäure), 2-Hydroxy-2-carboxypentan-1,5-disäure (Citronensäure), 2,3,4,5-Tetrahydroxyhexan-1,6-disäure (Isomere; Saccharinsäure, Schleimsäure etc.) bestehenden Gruppe.

9. Verwendung einer Zusammensetzung nach einem der Ansprüche 1-3, worin die verwandte Verbindung eine 2-Ketosäure der allgemeinen Formel
(R_{c}) CO COO (R_{d})
umfaßt, worin R_{c} und R_{d} gleich oder verschieden sein können und unabhängig voneinander unter H, einer Alkyl-, Aralkyl- oder Arylgruppe in gesättigter oder ungesättigter, isomerer oder nicht isomerer, geradkettiger oder verzweigtkettiger oder cyclischer Form mit 1 bis 29 Kohlenstoffatomen ausgewählt sind, und weiterhin R_{c} eine F-, Cl-, Br-, I-, OH-, CHO-, COOH- und Alkoxygruppe mit 1 bis 9 Kohlenstoffatomen tragen kann, wobei die genannte alpha-Ketosäure als freie Säure oder in Esterform oder in einer Salzform mit einer organischen Base oder einem anorganischen Alkali vorliegt.

10. Verwendung einer Zusammensetzung nach Anspruch 9, worin die 2-Ketosäure ausgewählt ist aus der aus 2-Ketoessigsäure (Glyoxylsäure), 2-Ketoessigsäuremethylester, 2-Ketopropansäure (Brenztraubensäure), 2-Ketopropansäuremethylester (Methylpyruvat), 2-Ketopropansäureethylester (Ethylpyruvate), 2-Ketopropansäurepropylester (Propylpyruvat), 2-Phenyl-2-ketoessigsäure (Benzoylameisensäure), 2-Phenyl-2-ketoessigsäuremethylester (Benzoylameisensäuremethylester), 2-Phenyl-2-ketoessigsäureethylester (Benzoylameisensäureethylester), 3-Phenyl-2-ketopropansäure (Phenylbrenztraubensäure), 3-Phenyl-2-ketopropansäuremethylester (Phenylbrenztraubensäuremethylester), 3-Phenyl-2-ketopropansäureethylester (Phenylbrenztraubensäureethylester), 2-Ketobutansäure, 2-Ketopentansäure, 2-Ketohexansäure, 2-Ketoheptansäure, 2-Ketooctansäure, 2-Ketododecansäure, und 2-Ketooctansäuremethylester bestehenden Gruppe.

11. Verwendung einer Zusammensetzung nach Anspruch 10, worin die genannte verwandte Verbindung ein 2-Ketocarbonsäureester ist.

12. Verwendung einer Zusammensetzung nach einem der Ansprüche 1-3, worin die genannte 2-Hydroxycarbonsäure oder verwandte Verbindung ausgewählt ist aus der aus Isocitronensäure, Methylmilchsäure, Mandelsäure, Chinasäure oder Chinolacton, Äpfelsäure, Brenztraubensäureethylester (Ethylpyruvat), Tropasäure, Ascorbinsäure oder Ascorbolacton, Benzilsäure, Ribonsäure oder Ribonolacton, Saccharinsäure oder Saccharolacton, Schleimsäure oder Mucolacton, Citramalsäure, Galactonsäure oder Galactonolacton, Gulonsäure oder Gulonlacton, Glucoheptonsäure oder Glucoheptonlacton, Pantoinsäure oder Pantolacton, Phenylmilchsäure, Gluconsäure oder Gluconolacton bestehenden Gruppe.

13. Verwendung einer oder mehrerer 2-Hydroxycarbonsäuren, 2-Ketosäuren oder verwandter Verbindungen, wobei die genannten verwandten Verbindungen ausgewählt sind aus der aus Ascorbinsäure, Chinasäure, Isocitronensäure, Tropasäure, Trethocansäure, 3-Chlormilchsäure, Cerebronsäure, Citramalsäure, Agaricinsäure, Aleuritinsäure, Pantoinsäure, Lactobionsäure und Hexulosonsäure bestehenden Gruppe, zusammen mit einem Träger, der für die topische Anwendung auf menschlichen oder tierischen Nägeln geeignet ist, für die Herstellung einer therapeutischen Zusammensetzung zur Linderung von Anzeichen von Nägelveränderungen, die mit intrinsischem und/oder extrinsischem Altern verbunden sind.

14. Verwendung nach Anspruch 13, worin das genannte extrinsische Altern Sonnenlicht, Bestrahlungen, Luftverschmutzung, Wind, Kälte, Feuchtigkeit, Wärme oder Hitze, Chemikalien, Rauch, Zigarettenrauchen, und zwar eines oder mehrere unter den vorstehend genannten, einschließt.

15. Verwendung nach Anspruch 13 oder 14, worin die Anzeichen von Nägelveränderungen, die mit intrinsischem und/oder extrinsischem Altern verbunden sind, das Dünnerwerden, Brüchigwerden, Splittern, Glanzverlust, unebene Oberfläche, Biegsamkeitsverlust, Elastizitätsverlust und Verlust der Spannkraft umfassen.

16. Verwendung nach einem der Ansprüche 13-15, worin die genannte 2-Hydroxycarbonsäure durch eine allgemeine Strukturformel dargestellt werden kann:
(Rₐ) (R_{b}) C (OH) COOH
worin Rₐ und R_{b} gleich oder verschieden sein können und unabhängig voneinander ausgewählt sind unter H, F, Cl, Br, Alkyl-, Aralkyl- or Arylgruppen in gesättigter oder ungesättigter, isomerer oder nicht isomerer, geradkettiger oder verzweigtkettiger oder cyclischer Form mit 1 bis 29 Kohlenstoffatomen, und weiterhin Rₐ und R_{b} eine OH-, CHO-, COOH- und Alkoxygruppe mit 1 bis 9 Kohlenstoffatomen tragen können, wobei die genannte 2-Hydroxycarbonsäure als freie Säure oder in Lactonform oder in Salzform mit einer organischen Base oder einem anorganischen Alkali vorliegen kann, sowie als Stereoisomere in D-, L- und DL-Form, wenn Rₐ und R_{b} nicht identisch sind.

17. Verwendung nach Anspruch 16, worin die genannte 2-Hydroxycarbonsäure in Salz- oder Lactonform vorliegt, vorzugsweise ausgewählt aus der aus Gluconolacton, Galactonolacton, Glucuronlacton, Galacturonlacton, Gulonlacton, Ribonolacton, Saccharinsäurelacton, Pantoyllacton, Glucoheptonlacton, Mannonlacton und Galactoheptonlacton bestehenden Gruppe.

18. Verwendung nach Anspruch 16, worin die genannte 2-Hydroxycarbonsäure eine Alkylhydroxycarbonsäure ist, vorzugsweise ausgewählt aus der aus 2-Hydroxyessigsäure (Glycolsäure), 2-Hydroxypropansäure (Milchsäure), 2-Methyl-2-hydroxypropansäure (Methylmilchsäure), 2-Hydroxybutansäure, 2-Hydroxypentansäure, 2-Hydroxyhexansäure, 2-Hydroxyheptansäure, 2-Hydroxyoctansäure, 2-Hydroxynonansäure, 2-Hydroxydecansäure, 2-Hydroxyundecansäure, 2-Hydroxydodecansäure (alpha-Hydroxylaurinsäure), 2-Hydroxytetradecansäure (alpha-Hydroxymyristinsäure), 2-Hydroxyhexadecansäure (alpha-Hydroxypalmitinsäure), 2-Hydroxyoctadecansäure (alpha-Hydroxystearinsäure), 2-Hydroxyeicosansäure (alpha-Hydroxyarachidonsäure), 2-Hydroxytetraeicosansäure (Cerebronsäure) und 2-Hydroxytetraeicosensäure (alpha-Hydroxynervonsäure) bestehenden Gruppe.

19. Verwendung nach Anspruch 16, worin die genannte 2-Hydroxycarbonsäure eine Aralkyl- or Aryl-2-hydroxycarbonsäure ist, vorzugsweise ausgewählt aus der aus 2-Phenyl-2-hydroxyessigsäure (Mandelsäure), 2,2-Diphenyl-2-hydroxyessigsäure (Benzilsäure), 3-Phenyl-2-hydroxypropansäure (Phenylmilchsäure), 2-Phenyl-2-methyl-2-hydroxyessigsäure (Atrolactinsäure), 2-(4'-Hydroxyphenyl)-2-hydroxyessigsäure, 2-(4'-Chlorphenyl)-2-hydroxyessigsäure, 2-(3'-Hydroxy-4'-methoxyphenyl)-2-hydroxyessigsäure, 2-(4'-Hydroxy-3'-methoxyphenyl)-2-hydroxyessigsäure, 3-(2'-Hydroxyphenyl)-2-hydroxypropansäure, 3-(4'-Hydroxyphenyl)-2-hydroxypropansäure und 2-(3',4'-Dihydroxyphenyl)-2-hydroxyessigsäure bestehenden Gruppe.

20. Verwendung nach Anspruch 16, worin die genannte 2-Hydroxycarbonsäure eine Polyhydroxycarbonsäure oder Hydroxypolycarbonsäure ist, vorzugsweise ausgewählt aus der aus 2,3-Dihydroxypropansäure (Glycerinsäure), 2,3,4-Trihydroxybutansäure (Isomere; Erythronsäure, Threonsäure), 2,3,4,5-Tetrahydroxypentansäure (Isomere; Ribonsäure, Arabinsäure, Xylonsäure, Lyxonsäure), 2,3,4,5,6-Pentahydroxyhexansäure (Isomere; Allonsäure, Altronsäure, Gluconsäure, Mannonsäure, Gulonsäure, Idonsäure, Galactonsäure, Talonsäure), 2,3,4,5,6,7-Hexahydroxyheptansäure (Isomere; Glucoheptonsäure, Galactoheptonsäure etc.) und Hydroxypropan-1,3-disäure (Tartronsäure), 2-Hydroxybutan-1,4-disäure (Äpfelsäure), 2,3-Dihydroxybutan-1,4-disäure (Weinsäure), 2-Hydroxy-2-carboxypentan-1,5-disäure (Citronensäure), 2,3,4,5-Tetrahydroxyhexan-1,6-disäure (Isomere; Saccharinsäure, Schleimsäure etc.) bestehenden Gruppe.

21. Verwendung nach einem der Ansprüche 13-15, worin die verwandte Verbindung eine 2-Ketosäure der allgemeinen Formel
(R_{c}) CO COO (R_{d})
umfaßt, worin R_{c} und R_{d} gleich oder verschieden sein können und unabhängig voneinander unter H, einer Alkyl-, Aralkyl- oder Arylgruppe in gesättigter oder ungesättigter, isomerer oder nicht isomerer, geradkettiger oder verzweigtkettiger oder cyclischer Form mit 1 bis 29 Kohlenstoffatomen ausgewählt sind, und weiterhin R_{c} eine F-, Cl-, Br-, I-, OH-, CHO-, COOH- und Alkoxygruppe mit 1 bis 9 Kohlenstoffatomen tragen kann, wobei die genannte alpha-Ketosäure als freie Säure oder in Esterform oder in einer Salzform mit einer organischen Base oder einem anorganischen Alkali vorliegt.

22. Verwendung nach Anspruch 21, worin die 2-Ketosäure ausgewählt ist aus der aus 2-Ketoessigsäure (Glyoxylsäure), 2-Ketoessigsäuremethylester, 2-Ketopropansäure (Brenztraubensäure), 2-Ketopropansäuremethylester (Methylpyruvat), 2-Ketopropansäureethylester (Ethylpyruvate), 2-Ketopropansäurepropylester (Propylpyruvat), 2-Phenyl-2-ketoessigsäure (Benzoylameisensäure), 2-Phenyl-2-ketoessigsäuremethylester (Benzoylameisensäuremethylester), 2-Phenyl-2-ketoessigsäureethylester (Benzoylameisensäureethylester), 3-Phenyl-2-ketopropansäure (Phenylbrenztraubensäure), 3-Phenyl-2-ketopropansäuremethylester (Phenylbrenztraubensäuremethylester), 3-Phenyl-2-ketopropansäureethylester (Phenylbrenztraubensäureethylester), 2-Ketobutansäure, 2-Ketopentansäure, 2-Ketohexansäure, 2-Ketoheptansäure, 2-Ketooctansäure, 2-Ketododecansäure, und 2-Ketooctansäuremethylester bestehenden Gruppe.

23. Verwendung nach Anspruch 22, worin die genannte verwandte Verbindung ein 2-Ketocarbonsäureester ist.

24. Verwendung nach einem der Ansprüche 13-15, worin die genannte 2-Hydroxycarbonsäure oder verwandte Verbindung ausgewählt ist aus der aus Isocitronensäure, Methylmilchsäure, Mandelsäure, Chinasäure oder Chinolacton, Äpfelsäure, Brenztraubensäureethylester (Ethylpyruvat), Tropasäure, Ascorbinsäure oder Ascorbolacton, Benzilsäure, Ribonsäure oder Ribonolacton, Saccharinsäure oder Saccharolacton, Schleimsäure oder Mucolacton, Citramalsäure, Galactonsäure oder Galactonolacton, Gulonsäure oder Gulonlacton, Glucoheptonsäure oder Glucoheptonlacton, Pantoinsäure oder Pantolacton, Phenylmilchsäure, Gluconsäure oder Gluconolacton bestehenden Gruppe.

25. Verwendung einer Zusammensetzung nach einem der Ansprüche 1 bis 4, worin die genannte 2-Hydroxycarbonsäure, 2-Ketosäure oder verwandte Verbindung 2-Hydroxyessigsäure (Glycolsäure) ist.

26. Verwendung einer Zusammensetzung nach einem der Ansprüche 1 bis 4, worin die genannte 2-Hydroxycarbonsäure, 2-Ketosäure oder verwandte Verbindung 2-Hydroxypropansäure (Milchsäure) ist.

27. Verwendung einer Zusammensetzung nach einem der Ansprüche 1 bis 4, worin die genannte 2-Hydroxycarbonsäure, 2-Ketosäure oder verwandte Verbindung 2-Hydroxy-2-carboxypentane-1,5-disäure (Citronensäure) ist.

28. Verwendung einer Zusammensetzung nach einem der Ansprüche 1 bis 4, worin die genannte 2-Hydroxycarbonsäure, 2-Ketosäure oder verwandte Verbindung 2-Methyl-2-hydroxypropansäure (Methylmilchsäure) ist.

29. Verwendung einer Zusammensetzung nach einem der Ansprüche 1 bis 4, worin die genannte 2-Hydroxycarbonsäure, 2-Ketosäure oder verwandte Verbindung Gluconolacton ist.

30. Verwendung einer Zusammensetzung nach einem der Ansprüche 1 bis 4, worin die genannte 2-Hydroxycarbonsäure, 2-Ketosäure oder verwandte Verbindung Chinasäure, Ribonolacton, Äpfelsäure, Mandelsäure, Benzilsäure, Galactonolacton, Ascorbinsäure, Brenztraubensäureethylester oder Tropasäure ist.

31. Verwendung nach einem der Ansprüche 13-17, worin die genannte 2-Hydroxycarbonsäure, 2-Ketosäure oder verwandte Verbindung 2-Hydroxyessigsäure (Glycolsäure) ist.

32. Verwendung nach einem der Ansprüche 13-17, worin die genannte 2-Hydroxycarbonsäure, 2-Ketosäure oder verwandte Verbindung 2-Hydroxypropansäure (Milchsäure) ist.

33. Verwendung nach einem der Ansprüche 13-17, worin die genannte 2-Hydroxycarbonsäure, 2-Ketosäure oder verwandte Verbindung 2-Hydroxy-2-carboxypentane-1,5-disäure (Citronensäure) ist.

34. Verwendung nach einem der Ansprüche 13-17, worin die genannte 2-Hydroxycarbonsäure, 2-Ketosäure oder verwandte Verbindung 2-Methyl-2-hydroxypropansäure (Methylmilchsäure) ist.

35. Verwendung nach einem der Ansprüche 13-17, worin die genannte 2-Hydroxycarbonsäure, 2-Ketosäure oder verwandte Verbindung Gluconolacton ist.

36. Verwendung nach einem der Ansprüche 13-17, worin die genannte 2-Hydroxycarbonsäure, 2-Ketosäure oder verwandte Verbindung Chinasäure, Ribonolacton, Äpfelsäure, Mandelsäure, Benzilsäure, Galactonolacton, Ascorbinsäure, Brenztraubensäureethylester oder Tropasäure ist.

## Revendications

1. Utilisation d'une composition cosmétique comprenant un ou plusieurs acides 2-hydroxycarboxyliques, 2-cétoacides ou composés apparentés, lesdits composés apparentés étant choisis dans le groupe consistant en acide ascorbique, acide quinique, acide isocitrique, acide tropique, acide tréthocanique, acide 3-chlorolactique, acide cérébronique, acide citramalique, acide agaricique, acide aleuritique, acide pantoïque, acide lactobionique, et acide hexulosonique, dans un véhicule acceptable du point de vue topique pour l'application topique aux ongles de l'homme ou d'animaux, pour atténuer les signes de modifications des ongles associés à un vieillissement intrinsèque et/ou extrinsèque.

2. Utilisation d'une composition suivant la revendication 1, dans laquelle le vieillissement extrinsèque comprend une ou plusieurs quelconques des actions de la lumière solaire, de rayonnements, de la pollution de l'air, du vent, du froid, de l'humidité, de la chaleur, d'agents chimiques, de la fumée et du tabagisme.

3. Utilisation d'une composition suivant l'une quelconque des revendications 1 et 2, dans laquelle les signes de modifications des ongles associés à un vieillissement intrinsèque et/ou extrinsèque comprennent un amincissement, une fragilité, un dédoublement, une absence d'éclat, une surface irrégulière, une perte de flexibilité, une perte d'élasticité et une perte de résilience.

4. Utilisation d'une composition suivant l'une quelconque des revendications 1 à 3, dans laquelle l'acide 2-hydroxycarboxylique peut être représenté par une structure générale de formule :
(Rₐ) (R_{b}) C (OH) COOH
dans laquelle Rₐ et R_{b} peuvent être identiques ou différents et sont choisis indépendamment entre H, F, Cl, Br, des groupes alkyle, aralkyle ou aryle saturés ou insaturés, isomères ou non isomères, à chaîne droite ou à chaîne ramifiée ou de forme cyclique, ayant 1 à 29 atomes de carbone et, en outre, Rₐ et R_{b} peuvent porter des groupes OH, CHO, COOH et alkoxy ayant 1 à 9 atomes de carbone, ledit acide 2-hydroxycarboxylique peut être présent sous forme d'un acide libre ou sous forme d'une lactone ou sous forme d'un sel formé avec une base organique ou une substance alcaline inorganique, et sous forme de stéréo-isomères consistant en les formes D, L et DL lorsque Rₐ et R_{b} ne sont pas identiques.

5. Utilisation d'une composition suivant la revendication 4, dans laquelle l'acide 2-hydroxycarboxylique est sous forme d'un sel ou d'une lactone, choisie de préférence dans le groupe consistant en gluconolactone, galactonolactone, glucuronolactone, galactrononolactone, gulonolactone, ribonolactone, lactone d'acide saccharique, pantoyllactone, glucoheptonolactone, mannonolactone et galactoheptonolactone.

6. Utilisation d'une composition suivant la revendication 4, dans laquelle l'acide 2-hydroxycarboxylique est un acide alkylhydroxycarboxylique, choisi de préférence dans le groupe consistant en l'acide 2-hydroxyéthanoïque (acide glycolique), l'acide 2-hydroxypropanoïque (acide lactique), l'acide 2-méthyl-2-hydroxypropanoïque (acide méthyllactique), l'acide 2-hydroxybutanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxynonanoïque, l'acide 2-hydroxydécanoïque, l'acide 2-hydroxy-undécanoïque, l'acide 2-hydroxydodécanoïque (acide alpha-hydroxylaurique), l'acide 2-hydroxytétradécanoïque (acide alpha-hydroxymyristique), l'acide 2-hydroxyhexadécanoïque (acide alpha-hydroxypalmitique), l'acide 2-hydroxyoctadénanoïque (acide alpha-hydroxystéarique), l'acide 2-hydroxyeicosanoïque (acide alpha-hydroxyarachidonique), l'acide 2-hydroxytétra-eicosanoïque (acide cérébronique) et l'acide 2-hydroxytétra-eicosénoïque (acide alpha-hydroxynervonique).

7. Utilisation d'une composition suivant la revendication 4, dans laquelle l'acide 2-hydroxycarboxylique est un acide aralkyl- ou aryl-2-hydroxycarboxylique, choisi de préférence dans le groupe consistant en l'acide 2-phényl-2-hydroxyéthanoïque (acide mandélique), l'acide 2,2-diphényl-2-hydroxyéthanoïque (acide benzilique), l'acide 3-phényl-2-hydroxypropanoïque (acide phényllactique), l'acide 2-phényl-2-méthyl-2-hydroxyéthanoïque (acide atrolactique), l'acide 2-(4'-hydroxyphényl)2-hydroxyéthanoïque, l'acide 2-(4'-chlorophényl)2-hydroxyéthanoïque, l'acide 2-(3'-hydroxy-4'-méthoxyphényl)2-hydroxyéthanoïque, l'acide 2-(4'-hydroxy-3'-méthoxyphényl)2-hydroxyéthanoïque, l'acide 3-(2'-hydroxyphényl)2-hydroxypropanoïque, l'acide 3-(4'-hydroxyphényl)2-hydroxypropanoïque et l'acide 2-(3',4'-dihydroxyphényl)2-hydroxyéthanoïque.

8. Utilisation d'une composition suivant la revendication 4, dans laquelle l'acide 2-hydroxycarboxylique est un acide polyhydroxycarboxylique ou un acide hydroxypolycarboxylique, choisi de préférence dans le groupe consistant en l'acide 2,3-dihydroxypropanoïque (acide glycérique), l'acide 2,3,4-trihydroxybutanoïque (isomères ; acide érythronique, acide thréonique), l'acide 2,3,4,5-tétrahydroxypentanoïque (isomères ; acide ribonique, acide arabinoïque, acide xylonique, acide lyxonique), l'acide 2,3,4,5,6-pentahydroxyhexanoïque (isomères ; acide allonique, acide altronique, acide gluconique, acide mannoïque, acide gulonique, acide idonique, acide galactonique, acide talonique), l'acide 2,3,4,5,6,7-hexahydroxyheptanoïque (isomères ; acide glucoheptonique, acide galactoheptonique, etc.) et l'acide hydroxy-propane-1,3-dioïque (acide tartronique), l'acide 2-hydroxybutane-1,4-dioïque (acide malique), l'acide 2,3-dihydroxy-butane-1,4-dioïque (acide tartrique), l'acide 2-hydroxy-2-carboxypentane-1,5-dioïque (acide citrique) et l'acide 2,3,4,5-tétrahydroxyhexane-1,6-dioïque (isomères ; acide saccharique, acide mucique, etc.).

9. Utilisation d'une composition suivant l'une quelconque des revendications 1 à 3, dans laquelle le composé apparenté comprend un 2-cétoacide de formule générale :
(R_{c}) CO COO (R_{d})
dans laquelle R_{c} et R_{d} peuvent être identiques ou différents et sont choisis indépendamment entre H et un groupe alkyle, aralkyle ou aryle saturé ou insaturé, isomère ou non isomère, à chaîne droite ou à chaîne ramifiée ou bien sous forme cyclique, ayant 1 à 29 atomes de carbone et, en outre, R_{c} peut porter F, Cl, Br, I, des groupes OH, CHO, COOH et alkoxy ayant 1 à 9 atomes de carbone, ledit alpha-cétoacide existant sous forme d'un acide libre ou sous forme d'un ester, ou bien sous forme d'un sel formé avec une base organique ou une substance alcaline inorganique.

10. Utilisation d'une composition suivant la revendication 9, dans laquelle le 2-cétoacide est choisi dans le groupe consistant en l'acide 2-cétoéthanoïque (acide glyoxylique), le 2-cétoéthanoate de méthyle, l'acide 2-cétopropanoïque (acide pyruvique), le 2-cétopropanoate de méthyle (pyruvate de méthyle), le 2-cétopropanoate d'éthyle (pyruvate d'éthyle), le 2-cétopropanoate de propyle (pyruvate de propyle), l'acide 2-phényl-2-cétoéthanoïque (acide benzoylformique), le 2-phényl-2-cétoéthanoate de méthyle, (benzoylformiate de méthyle), le 2-phényl-2-cétoéthanoate d'éthyle (benzoylformiate d'éthyle), l'acide 3-phényl-2-cétopropanoïque (acide phénylpyruvique), le 3-phényl-2-cétopropanoate de méthyle (phénylpyruvate de méthyle), le 3-phényl-2-cétopropanoate d'éthyle (phénylpyruvate d'éthyle), l'acide 2-cétobutanoïque, l'acide 2-cétopentanoïque, l'acide 2-cétohexanoïque, l'acide 2-cétoheptanoïque, l'acide 2-cétooctanoïque, l' acide 2-cétododécanoïque et le 2-céto-octanoate de méthyle.

11. Utilisation d'une composition suivant la revendication 10, dans laquelle le composé apparenté est un ester d'acide 2-cétocarboxylique.

12. Utilisation d'une composition suivant l'une quelconque des revendications 1 à 3, dans laquelle l'acide 2-hydroxycarboxylique ou le composé apparenté est choisi dans le groupe consistant en l'acide isocitrique, l'acide méthyllactique, l'acide mandélique, l'acide quinique ou la quinolactone, l'acide malique, le pyruvate d'éthyle, l'acide tropique, l'acide ascorbique ou l'ascorbolactone, l'acide benzilique, l'acide ribonique ou la ribonolactone, l'acide saccharique ou la saccharolactone, l'acide mucique ou la mucolactone, l'acide citramalique, l'acide galactonique ou la galactonolactone, l'acide gulonique ou la gulonolactone, l'acide glucoheptonique ou la glucoheptonolactone, l'acide pantoïque ou la pantolactone, l'acide phényllactique, l'acide gluconique ou la gluconolactone.

13. Utilisation d'un ou plusieurs acides 2-hydroxycarboxyliques, 2-cétoacides ou composés apparentés, lesdits composés apparentés étant choisis dans le groupe consistant en l'acide ascorbique, l'acide quinique, l'acide isocitrique, l'acide tropique, l'acide tréthocanique, l'acide 3-chlorolactique, l'acide cérébronique, l'acide citramalique, l'acide agaricique, l'acide aleuritique, l'acide pantoïque, l'acide lactobionique et l'acide hexulosonique, conjointement avec un véhicule acceptable pour l'application topique aux ongles de l'homme ou d'animaux, pour la préparation d'une composition thérapeutique destinée à atténuer les signes de modifications des ongles associés à un vieillissement intrinsèque et/ou extrinsèque.

14. Utilisation suivant la revendication 13, dans laquelle le vieillissement extrinsèque comprend un ou plusieurs effets quelconques de la lumière solaire, de rayonnements, de la pollution de l'air, du vent, du froid, de l'humidité, de la chaleur, d'agents chimiques, de la fumée, du tabagisme.

15. Utilisation suivant l'une quelconque des revendications 13 et 14, dans laquelle les signes de modification des ongles associés à un vieillissement intrinsèque et/ou extrinsèque comprennent un amincissement, une fragilité, un dédoublement, un manque d'éclat, une surface irrégulière, une perte de flexibilité, une perte d'élasticité et une perte de résilience.

16. Utilisation suivant l'une quelconque des revendications 13 à 15, dans laquelle l'acide 2-hydroxycarboxylique peut être représenté par une structure générale de formule :
(Rₐ) (R_{b}) C (OH) COOH
dans laquelle Rₐ et R_{b} peuvent être identiques ou différents et sont choisis indépendamment entre H, F, Cl, Br, des groupes alkyle, aralkyle ou aryle saturés ou insaturés, isomères ou non isomères, à chaîne droite ou à chaîne ramifiée ou de forme cyclique, ayant 1 à 29 atomes de carbone et, en outre, Rₐ et R_{b} peuvent porter des groupes OH, CHO, COOH et alkoxy ayant 1 à 9 atomes de carbone, ledit acide 2-hydroxycarboxylique peut être présent sous forme d'un acide libre ou sous forme d'une lactone ou sous forme d'un sel formé avec une base organique ou une substance alcaline inorganique, et sous forme de stéréo-isomères consistant en les formes D, L et DL lorsque Rₐ et R_{b} ne sont pas identiques.

17. Utilisation suivant la revendication 16, dans laquelle l'acide 2-hydroxycarboxylique est sous forme d'un sel ou sous forme d'une lactone, choisie de préférence dans le groupe consistant en gluconolactone, galactonolactone, glucuronolactone, galactrononolactone, gulonolactone, ribonolactone, lactone d'acide saccharique, pantoyllactone, glucoheptonolactone, mannonolactone et galactoheptonolactone.

18. Utilisation suivant la revendication 16, dans laquelle l'acide 2-hydroxycarboxylique est un acide alkylhydroxycarboxylique choisi de préférence dans le groupe consistant en l'acide 2-hydroxyéthanoïque (acide glycolique), l'acide 2-hydroxypropanoïque (acide lactique), l'acide 2-méthyl-2-hydroxypropanoïque (acide méthyllactique), l'acide 2-hydroxybutanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxynonanoïque, l'acide 2-hydroxydécanoïque, l'acide 2-hydroxyundécanoïque, l'acide 2-hydroxydodécanoïque (acide alpha-hydroxylaurique), l'acide 2-hydroxytétradécanoïque (acide alpha-hydroxymyristique), l'acide 2-hydroxyhexadécanoïque (acide alpha-hydroxypalmitique), l'acide 2-hydroxyoctadécanoïque (acide alpha-hydroxystéarique), l'acide 2-hydroxyeicosanoïque (acide alpha-hydroxy-arachidonique), l'acide 2-hydroxytétraeicosanoïque (acide cérébronique) et l'acide 2-hydroxytétraeicosénoïque (acide alpha-hydroxynervonique).

19. Utilisation suivant la revendication 16, dans laquelle l'acide 2-hydroxycarboxylique est un acide aralkyl- ou aryl-2-hydroxycarboxylique, choisi de préférence dans le groupe consistant en l'acide 2-phényl-2-hydroxyéthanoïque (acide mandélique), l'acide 2,2-diphényl-2-hydroxyéthanoïque (acide benzilique), l'acide 3-phényl-2-hydroxypropanoïque (acide phényllactique), l'acide 2-phényl-2-méthyl-2-hydroxyéthanoïque (acide atrolactique), l'acide 2-(4'-hydroxyphényl)2-hydroxyéthanoïque, l'acide 2-(4'-chlorophényl)-2-hydroxyéthanoïque, l'acide 2-(3'-hydroxy-4'-méthoxy-phényl)-2-hydroxyéthanoïque, l'acide 2-(4'-hydroxy-3'-méthoxyphényl)-2-hydroxyéthanoïque, l'acide 3-(2'-hydroxyphényl)-2-hydroxypropanoïque, l'acide 3-(4'-hydroxyphényl)2-hydroxypropanoïque et l'acide 2-(3',4'-dihydroxyphényl)2-hydroxyéthanoïque.

20. Utilisation suivant la revendication 16, dans laquelle l'acide 2-hydroxycarboxylique est un acide polyhydroxycarboxylique ou un acide hydroxypolycarboxylique, choisi de préférence dans le groupe consistant en l'acide 2,3-dihydroxypropanoïque (acide glycérique), l'acide 2,3,4-trihydroxybutanoïque (isomères ; acide érythronique, acide thréonique), l'acide 2,3,4,5-tétrahydroxy-pentanoïque (isomères ; acide ribonique, acide arabinoïque, acide xylonique, acide lyxonique), l'acide 2,3,4,5,6-penta-hydroxyhexanoïque (isomères ; acide allonique, acide altronique, acide gluconique, acide mannoïque, acide gulonique, acide idonique, acide galactonique, acide talonique), l'acide 2,3,4,5,6,7-hexahydroxyheptanoïque (isomères ; acide glucoheptonique, acide galactoheptonique, etc.) et l'acide hydroxy-propane-1,3-dioïque (acide tartronique), l'acide 2-hydroxybutane-1,4-dioïque (acide malique), l'acide 2,3-dihydroxy-butane-1,4-dioïque (acide tartrique), l'acide 2-hydroxy-2-carboxypentane-1,5-dioïque (acide citrique) et l'acide 2,3,4,5-tétrahydroxyhexane-1,6-dioïque (isomères ; acide saccharique, acide mucique, etc.).

21. Utilisation suivant l'une quelconque des revendications 13 à 15, dans laquelle le composé apparenté comprend un 2-cétoacide de formule générale :
(R_{c}) CO COO (R_{d})
dans laquelle R_{c} et R_{d} peuvent être identiques ou différents et sont choisis indépendamment entre H et un groupe alkyle, aralkyle ou aryle saturé ou insaturé, isomère ou non isomère, à chaîne droite ou à chaîne ramifiée ou bien sous forme cyclique, ayant 1 à 29 atomes de carbone et, en outre, R_{c} peut porter F, Cl, Br, I, des groupes OH, CHO, COOH et alkoxy ayant 1 à 9 atomes de carbone, ledit alpha-cétoacide existant sous forme d'un acide libre ou sous forme d'un ester, ou bien sous forme d'un sel formé avec une base organique ou une substance alcaline inorganique.

22. Utilisation suivant la revendication 21, dans laquelle le 2-cétoacide est choisi dans le groupe consistant en l'acide 2-cétoéthanoïque (acide glyoxylique), le 2-cétoéthanoate de méthyle, l'acide 2-cétopropanoïque (acide pyruvique), le 2-cétopropanoate de méthyle (pyruvate de méthyle), le 2-cétopropanoate d'éthyle (pyruvate d'éthyle), le 2-cétopropanoate de propyle (pyruvate de propyle), l'acide 2-phényl-2-cétoéthanoïque (acide benzoylformique), le 2-phényl-2-cétoéthanoate de méthyle (benzoylformiate de méthyle), le 2-phényl-2-cétoéthanoate d'éthyle (benzoylformiate d'éthyle), l'acide 3-phényl-2-cétopropanoïque (acide phénylpyruvique), le 3-phényl-2-cétopropanoate de méthyle (phénylpyruvate de méthyle), le 3-phényl-2-cétopropanoate d'éthyle (phénylpyruvate d'éthyle), l'acide 2-cétobutanoïque, l'acide 2-cétopentanoïque, l'acide 2-cétohexanoïque, l'acide 2-cétoheptanoïque, l'acide 2-céto-octanoïque, l'acide 2-cétododécanoïque et le 2-céto-octanoate de méthyle.

23. Utilisation suivant la revendication 22, dans laquelle le composé apparenté est un ester d'acide 2-cétocarboxylique.

24. Utilisation suivant l'une quelconque des revendications 13 à 15, dans laquelle l'acide 2-hydroxycarboxylique ou le composé apparenté est choisi dans le groupe consistant en l'acide isocitrique, l'acide méthyllactique, l'acide mandélique, l'acide quinique ou la quinolactone, l'acide malique, le pyruvate d'éthyle, l'acide tropique, l'acide ascorbique ou l'ascorbolactone, l'acide benzilique, l'acide ribonique ou la ribonolactone, l'acide saccharique ou la saccharolactone, l'acide mucique ou la mucolactone, l'acide citramalique, l'acide galactonique ou la galactonolactone, l'acide gulonique ou la gulonolactone, l'acide glucoheptonique ou la glucoheptonolactone, l'acide pantoïque ou la pantolactone, l'acide phényllactique, l'acide gluconique ou la gluconolactone.

25. Utilisation d'une composition suivant l'une quelconque des revendications 1 à 4, dans laquelle l'acide 2-hydroxycarboxylique, le 2-cétoacide ou le composé apparenté consiste en l'acide 2-hydroxyéthanoïque (acide glycolique).

26. Utilisation d'une composition suivant l'une quelconque des revendications 1 à 4, dans laquelle l'acide 2-hydroxycarboxylique, le 2-cétoacide ou le composé apparenté consiste en l'acide 2-hydroxypropanoïque (acide lactique).

27. Utilisation d'une composition suivant l'une quelconque des revendications 1 à 4, dans laquelle l'acide 2-hydroxycarboxylique, le 2-cétoacide ou le composé apparenté consiste en l'acide 2-hydroxy-2-carboxypentane-1,5-dioïque (acide citrique).

28. Utilisation d'une composition suivant l'une quelconque des revendications 1 à 4, dans laquelle l'acide 2-hydroxycarboxylique, le 2-cétoacide ou le composé apparenté consiste en l'acide 2-méthyl-2-hydroxypropanoïque (acide méthyllactique).

29. Utilisation d'une composition suivant l'une quelconque des revendications 1 à 4, dans laquelle l'acide 2-hydroxycarboxylique, le 2-cétoacide ou le composé apparenté consiste en gluconolactone.

30. Utilisation d'une composition suivant l'une quelconque des revendications 1 à 4, dans laquelle l'acide 2-hydroxycarboxylique, le 2-cétoacide ou le composé apparenté consiste en l'acide quinique, la ribonolactone, l'acide malique, l'acide mandélique, l'acide benzilique, la galactonolactone, l'acide ascorbique, le pyruvate d'éthyle ou l'acide tropique.

31. Utilisation suivant l'une quelconque des revendications 13 à 17, dans laquelle l'acide 2-hydroxycarboxylique, le 2-cétoacide ou le composé apparenté consiste en l'acide 2-hydroxyéthanoïque (acide glycolique).

32. Utilisation suivant l'une quelconque des revendications 13 à 17, dans laquelle l'acide 2-hydroxycarboxylique, le 2-cétoacide ou le composé apparenté consiste en l'acide 2-hydroxypropanoïque (acide lactique).

33. Utilisation suivant l'une quelconque des revendications 13 à 17, dans laquelle l'acide 2-hydroxycarboxylique, le 2-cétoacide ou le composé apparenté consiste en l'acide 2-hydroxy-2-carboxypentane-1,5-dioïque (acide citrique).

34. Utilisation suivant l'une quelconque des revendications 13 à 17, dans laquelle l'acide 2-hydroxycarboxylique, le 2-cétoacide ou le composé apparenté consiste en l'acide 2-méthyl-2-hydroxypropanoïque (acide méthyllactique).

35. Utilisation suivant l'une quelconque des revendications 13 à 17, dans laquelle l'acide 2-hydroxycarboxylique, le 2-cétoacide ou le composé apparenté consiste en la gluconolactone.

36. Utilisation suivant l'une quelconque des revendications 13 à 17, dans laquelle l'acide 2-hydroxycarboxylique, le 2-cétoacide ou le composé apparenté consiste en l'acide quinique, la ribonolactone, l'acide malique, l'acide mandélique, l'acide benzilique, la galactolactone, l'acide ascorbique, le pyruvate d'éthyle ou l'acide tropique.
